# EUROPEAN PATENT APPLICATION

(11) **EP 4 647 496 A1**
(43) Date of publication of application: **12.11.2025**
(21) Application number: 24731453.7
(22) Date of filing: 17.05.2024
(51) Int. Cl.: C12N 15/63

(54) **NOVEL PROMOTER AND USE THEREOF**

(30) Priority: 15.03.2024 KR 20240036383; 17.04.2024 KR 20240051425
(71) Applicant: CJ CheilJedang Corporation, Seoul 04560 (KR)
(72) Inventor: KIM, Gyuree, Seoul 04560 (KR); EOM, Ga Eul, Seoul 04560 (KR); KIM, Moonjung, Seoul 04560 (KR); BAE, Jee Yeon, Seoul 04560 (KR); SHIM, Jihyun, Seoul 04560 (KR)
(74) Representative: Reddie & Grose LLP
(86) International application number: PCT/KR2024/095782
(87) International publication number: WO 2025/192843

(57) **Abstract**

The present application relates to a novel promoter, and a method for preparing a target product using the same, and the polynucleotide according to one specific embodiment has a promoter activity, and is introduced into a microorganism, so it can increase expression and activity of a gene operatively linked thereto, and it can be usefully used for efficiently producing a target product affected by the polynucleotide and gene.

## Description

### [TECHNICAL FIELD]

### Cross-reference to prior application(s)

The present application claims the benefit of the priority based on Korean Patent Application No. 10-2024-0036383 filed on March 15, 2024 and Korean Patent Application No. 10-2024-0051425 filed on April 17, 2024, and the entire contents disclosed in the document of the corresponding Korean patent application are incorporated as a part of the present description.

The present application relates to a novel promoter, and a method for preparing a target product using thereof.

### [BACKGROUND ART]

In order to produce target substances such as amino acids or useful substances that can be used for various uses as feed, medicine, food and the like at high titer using microorganisms, efforts such as gene manipulation and/or foreign gene introduction and the like in the biosynthetic pathway have continued. As one of such methods, there is a method for inducing overexpression of a target gene in a microorganism, and for this, a high-efficiency gene expression system is needed. Since a promoter is one of elements involved in the gene expression level and expression regulation, development of a useful promoter is necessary for developing an expression system.

An E. coli-derived tac promoter is widely known as a strong promoter, and in case of coryneform microorganisms, strong promoters have been developed by modifying promoters of their own genes (Gene, 102, 93-98, 1991; Microbiology, 142, 1297-1309, 1996). On the other hand, the general structure of the promoter sequence for gene expression in the coryneform microorganisms is unknown, unlike other industrial microorganisms such as E. coli or Bacillus subtilis. Therefore, promoters have been developed, by measuring antibiotic resistance of a strain obtained by deleting the promoter part of an antibiotic resistant gene such as chloramphenicol, and cutting chromosomal DNA isolated from a coryneform microorganism with an appropriate restriction enzyme and introducing it thereto, and then transforming the coryneform microorganism with this. In addition, in order to overexpress a foreign gene in a Bacillus sp. microorganism, search for various promoters has been conducted so far, and production of enzymes for food, pharmaceuticals, and other industries using them has been conducted. Many vector systems using expression promoters of alpha-amylase, protease, and lipase genes in various Bacillus sp. microorganism have been made continuously up to now (Schumann 2007. Adv. Appl. Microbiol. 153:813-821).

However, a system which shows high expression efficiency in various microorganisms, that is, Escherichia sp. microorganisms, Corynebacterium sp. microorganisms, or Bacillus sp. microorganisms and the like, is still needed, so the necessity to develop a universal promoter is still emerging.

### [PRIOR ART]

### [Patent document] U.S. Granted Patent (US 11,041,181 B2)

### [DISCLOSURE]

### [TECHNICAL PROBLEM]

An object of the present application is to provide a novel polynucleotide.

Another object of the present application is to provide an expression cassette comprising the polynucleotide and a target gene.

Other object of the present application is to provide a recombinant vector (expression vector) comprising the polynucleotide or the expression cassette.

Other object of the present application is to provide a microorganism, comprising at least one selected from the group consisting of the polynucleotide, an expression cassette comprising the polynucleotide and a target gene, and a vector comprising the expression cassette.

Other object of the present application is to provide a method for producing a target product comprising culturing the microorganism in a medium. The method may further comprise recovering the target product from the medium or microorganism after the culturing.

Other object of the present application is to provide a composition for producing a target product comprising the microorganism, a medium in which the microorganism is cultured, or a combination thereof.

### [TECHNICAL SOLUTION]

Each description and embodiment disclosed in the present description can also be applied to each other description and embodiment. In other words, all combinations of the various elements disclosed herein belong to the scope of the present description. In addition, the scope of the present application cannot be considered limited by the specific description described below.

Hereinafter, it will be described in more detail.

One aspect of the present application provides a novel polynucleotide.

In the present description, "polynucleotide" comprises nucleotide monomers of 2 or more, 5 or more, 10 or more, 13 or more, 20 or more, or 30 or more, and the nucleotide monomers may be covalently bound to form a chain form.

In one embodiment of the present application, the polynucleotide may be a polynucleotide comprising any one nucleic acid sequence selected from the group consisting of SEQ ID NO: 1, SEQ ID NO: 2 and SEQ ID NO: 3.

In one embodiment, the polynucleotide may comprise the nucleic acid sequence of SEQ ID NO: 1.

In one embodiment, the polynucleotide may comprise the nucleic acid sequence of SEQ ID NO: 2.

In one embodiment, the polynucleotide may comprise the nucleic acid sequence of SEQ ID NO: 3.

The polynucleotide may have promoter activity, and/or may be used as a universal promoter.

In one embodiment, the polynucleotide may have promoter activity for expression of microorganisms of Corynebacterium genus, microorganisms of Escherichia genus, and/or microorganisms of Bacillus genus.

The polynucleotide according to one specific embodiment may be used as a synthetic promoter having strong expression inducing activity, and for example, it may express a target gene in a microorganism of Corynebacterium genus, a microorganism of Escherichia genus, and/or a microorganism of Bacillus genus with exceptionally higher efficiency than the known Pcj7 (SEQ ID NO: 15, U.S. Granted Patent US 7662943 B2) promoter.

The polynucleotide according to one specific embodiment may be natural or non-natural, and for example, it may be non-natural, which is chemically or recombinantly synthesized.

In the present description, "promoter" may mean a DNA region which comprises a binding site to polymerase and allows to initiate transcription of target DNA in the downstream. The promoter may be located at the 5' region of the transcription initiation site. The promoter may be operatively and/or able to regulate (intensify or weaken expression) linked to the upstream (toward the 5' end) of the target DNA. For example, the promoter is linked in the forward direction to the 5' end of any gene to intensify (increase) expression of the gene, or is linked in the reverse direction to the 3' end of any gene to weaken (reduce) expression of the corresponding gene. When a promoter is introduced in the reverse direction at the 3' end of any gene, for example, downstream of the stop codon, preferably, between the stop codon and the upstream of the transcription terminator, it may weaken expression of the gene, as an RNA polymerase complex causes a collision during the transcription process, by inducing transcription in the opposite direction to the normal transcription direction of the corresponding gene.

The polymerase is also called RNA polymerase, or DNA dependent RNA polymerase, and may refer to an enzyme which synthesizes primary transcript RNA. The polymerase may be RNA polymerase of prokaryotic cells, or RNA polymerase of eukaryotic cells (for example, RNA polymerase I, RNA polymerase II, RNA polymerase III, RNA polymerase IV or RNA polymerase V, etc.).

In the present description, "target sequence" may be a "target gene" subjected to expression. In one embodiment, the target gene may be a gene encoding a target protein. The target protein may be a protein (for example, enzyme) involved in production of a target product.

In the present description, the term "target product" may mean a biologically active substance to be finally produced or regulate (increase or reduce) production using the polynucleotide provided in the present description, an expression cassette, an expression vector, and/or a recombinant cell, comprising the same, and for example, it may mean the target protein encoded by the target gene itself, and/or all biologically active substances produced as the target protein involves therein. The biologically active substances mean all substances which is produced or derived from an organism (for example, cell) or has a certain function in a living body or cell, and for example, it may be at least one selected from the group consisting of amino acids, amino acid derivatives, nucleic acids (adenine, thymine, guanine, cytosine, uracil, etc.), nucleic acid derivatives, vitamins (vitamin A (retinol), B (B1 (thiamine), B2 (riboflavin), B3 (niacin), B5 (pantothenic acid), B6 (pyridoxine), B7, B9 (folic acid), B12 (cobalamin), etc.), C (ascorbic acid), D (calciferol), E (tocopherol), K (phylloquinone), etc.), vitamin derivatives, proteins (proteins other than the target protein, for example, hormones, growth factors, cytokines, immunoglobulins (antibodies), antigen proteins, receptors, ligands, functional fragments (fragments having targeted functions) thereof, fusion proteins in which at least 2 kinds are fused, etc.), sugars (for example, monosaccharides (glyceraldehyde, dihydroxy acetone, erythrose, threose, erythrulose, arabinose, lyxose, ribose, xylose, ribulose, xylulose, deoxyribose, allose, altrose, galactose, glucose, gulose, idose, mannose, talose, fructose, psicose, sorbose, tagatose, fucose, fuculose, rhamnose, mannoheptulose, sedoheptulose, etc.), disaccharides (cellobiose, isomaltose, isomaltulose, lactose, lactulose, maltose, sucrose, trehalose, turanose, etc.), polysaccharides, etc.), sugar derivatives (sugar alcohol, galactosamine, glucosamine, sialic acid, N-acetylglucosamine, sulfoquinovose, ascorbic acid, mannitol, glucuronic acid, etc.), fatty acids (myristoleic acid, palmitoleic acid, sapienic acid, oleic acid, elaidic acid, vaccenic acid, linoleic acid, linolelaidic acid, arachidonic acid, eicosapentaenoic acid (EPA), erucic acid, docosahexaenoic acid (DHA), etc.), fatty acid derivatives, organic acids (lactic acid, citric acid, oxalic acid, uric acid, butyric acid, stearic acid, propionic acid, etc.), metabolites thereof (polyhydroxyalkanoate (PHA), etc.), precursors thereof, derivatives maintaining biological activity thereof, and the like, but is not limited thereto.

In one embodiment, when the target protein is involved in production of the target product, (1) the target protein may be a protein involved in at least one process or step of intracellular pathway (for example, biosynthesis, metabolism, bioconversion, etc.), intracellular transport, and/or exocytosis pathway of the target product, for example, at least one selected from the group consisting of synthetase, lyase, kinase, carboxylase (e.g., pyruvate carboxylase, etc.), reductase, oxidase, decarboxylase, dehydrogenase, dehydratase, transferase (e.g., transferase), epimerase, etc.), intermediate products, carrier proteins, membrane proteins (channels, etc.) and the like, but is not limited thereto, and
(2) the target gene may be a gene encoding the target protein described in (1) above.

The amino acid in the target product, may be a proteinogenic amino acid, or non-proteinogenic amino acid.

The proteinogenic amino acid may be at least one selected from the group consisting of arginine, histidine, lysine, aspartic acid, glutamic acid, serine, threonine, asparagine, glutamine, cysteine, glycine, proline, selenocysteine, pyrrolysine, alanine, valine, isoleucine, leucine, methionine, phenylalanine, tyrosine and tryptophan, and in addition, it may be an intermediate thereof.

The proteinogenic amino acid may be an L-amino acid, for example, at least one selected from the group consisting of L-arginine, L-histidine, L-lysine, L-aspartic acid, L-glutamic acid, L-serine, L-threonine, L-asparagine, L-glutamine, L-tyrosine, L-alanine, L-isoleucine, L-leucine, L-valine, L-phenylalanine, L-methionine, L-tryptophan, L-glycine, L-proline and L-cysteine, or an intermediate thereof, and specifically, it may be L-lysine, L-threonine, L-isoleucine, L-leucine, L-valine, L-arginine, or L-glutamic acid, but is not limited thereto. The intermediate may be for example, O-acetyl homoserine, which is an intermediate of L-methionine, but is not limited thereto.

The proteinogenic amino acid may be at least one selected from the group consisting of beta-alanine (β -alanine), gamma-aminobutyric acid (γ -Aminobutyric acid, GABA), 5-aminolevulinic acid (δ -Aminolevulinic acid), p-aminobenzoic acid (4-Aminobenzoic acid), alpha-aminobutyric acid (α -aminoisobutyric acid), dehydroalanine, cystathionine, lanthionine, djenkolic acid, diaminopimelic acid, Norvaline, norleucine, alloisoleucine, t-leucine (tert-Leucine), alpha-amino-n-heptanoic acid (α-amino-n-heptanoic acid), pipecolic acid, alpha, beta-diaminopropionic acid (α , β -diaminopropionic acid), alpha-gamma-diaminobutyric acid (α , γ -diaminobutyric acid), ornithine, allothreonine, homocysteine, homoserine (or isothreonine), O-acetyl homoserine, and the like, but is not limited thereto.

The amino acid may be a D-amino acid or L-amino acid.

In one specific embodiment, the target product may be at least one selected from the group consisting of threonine, O-acetyl homoserine and valine.

The polynucleotide having promoter activity of the present application according to one specific embodiment may regulate (for example, increase or reduce) expression of a target gene operatively linked thereto, production and/or activity of a target protein encoded by the target gene, and/or production and/or activity of a biologically active substance involved in production of the target protein, compared to a conventional promoter or cell-intrinsic promoter, in a cell.

The nucleic acid sequence of the polynucleotide having promoter activity of the present application may be modified by conventionally known mutagenesis, for example, direct evolution and site-directed mutagenesis and the like. In other words, the polynucleotide may comprise a nucleic acid sequence having homology or identity of 60% or more, 65% or more, 70% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more or 99.9% or more to the nucleic acid sequence of SEQ ID NO: 1, SEQ ID NO: 2 or SEQ ID NO: 3 while having the same or corresponding biological activity to the nucleic acid sequence of SEQ ID NO: 1, SEQ ID NO: 2 or SEQ ID NO: 3. In addition, as long as it is a polynucleotide having the substantially same or corresponding biological activity to the nucleic acid sequence of SEQ ID NO: 1, SEQ ID NO: 2 or SEQ ID NO: 3, which is a sequence having homology or identity to the nucleic acid sequence of SEQ ID NO: 1, SEQ ID NO: 2 or SEQ ID NO: 3, a polynucleotide having a base sequence in which some sequences are deleted, modified, substituted or added in the nucleic acid sequence of SEQ ID NO: 1, SEQ ID NO: 2 or SEQ ID NO: 3 may be also included in the range of the polynucleotide having promoter activity of the present application.

In the present description, the term "homology" means the degree of matching with a given nucleic acid sequence or amino acid sequence, and may be expressed as a percentage (%). For example, the homology may be determined by directly aligning sequence information between two polynucleotide molecules or two polypeptide molecules, for example, parameters such as score, identity and similarity and the like, using an easily available computer program aligning sequence information. The computer program may be BLAST (NCBI), CLC Main Workbench (CLC bio), MegAlignTM (DNASTAR Inc) and the like.

In one specific embodiment, the polynucleotide comprising a specific nucleic acid sequence provided in the present description may be interpreted to comprise not only the specific nucleic acid sequence or a nucleic acid sequence substantially equivalent thereto, but also a nucleic acid sequence complementary to the specific nucleic acid sequence. Specifically, the complementary polynucleotide may be hybridized at a Tm value appropriately adjustable by those skilled in the art according to the purpose, for example, at a Tm value of 55°C, 60°C, 63°C or 65°C, and be analyzed under conditions described later: such conditions are specifically described in known documents. For example, a condition in which polynucleotides with a high complementarity of 60% or more, 70% or more, 80% or more, 85% or more, 90% or more, 91% or more, 92% or more, 93% or more, 94% or more, 95% or more, 96% or more, 97% or more, 98% or more, 98% or more, 99.5% or more, or 99.9% or more are hybridized, and polynucleotides having a lower complementarity are not hybridized, or a condition of washing once, specifically, twice or three times, at a salt concentration and temperature corresponding to 60°C, 1x SSC(saline-sodium citrate buffer), and 0.1%(w/v) SDS (Sodium Dodecyl Sulfate); 60°C, 0.1x SSC, and 0.1% SDS; or 68°C, 0.1x SSC, and 0.1% SDS, which is a washing condition of common southern hybridization, and the like may be listed, but is not limited thereto. Hybridization requires that two nucleotides have complementary sequences, but mismatches between bases may be allowed depending on the stringency of hybridization. The term, "complementary" may be used to describe relationship between nucleotide bases capable of hybridizing to each other. For example, in case of DNA, adenosine is complementary to thymine, and cytosine is complementary to guanine. The suitable stringency to hybridize a polynucleotide depends on the length and degree of complementarity of the polynucleotide, and this is well known in the technical field related thereto (See Sambrook et al., supra,9.50-9.51, 11.7-11.8).

According to one specific embodiment, the polynucleotide may comprise the nucleic acid sequence of SEQ ID NO: 1, SEQ ID NO: 2 or SEQ ID NO: 3, and may be a polynucleotide consisting of the nucleic acid sequence of SEQ ID NO: 1, SEQ ID NO: 2 or SEQ ID NO: 3 or essentially comprising thereof.

In the present description, that a polynucleotide or polypeptide "comprises a specific nucleic acid sequence or amino acid sequence" may refer to that the polynucleotide or polypeptide consists of the specific nucleic acid sequence or amino acid sequence or essentially comprises thereof, and it may be interpreted to include "a substantially equivalent sequence" in which a mutation (deletion, substitution, modification, and/or addition) is applied to the specific nucleic acid sequence or amino acid sequence within a range of maintaining an original function and/or desired function of the polynucleotide or polypeptide (or not exclude the mutation). In one embodiment, the nucleic acid sequence or amino acid sequence provided in the present description may include those modified by common mutagenesis, for example, direct evolution and/or site-directed mutagenesis and the like in a range of maintaining an original function or desired function thereof. In one specific embodiment, that a polynucleotide or polypeptide "comprises a specific nucleic acid sequence or amino acid sequence" may refer to that the polynucleotide or polypeptide (i) consists of the specific nucleic acid sequence or amino acid sequence, or essentially comprises thereof, or (ii) consists of an amino acid sequence having homology or identity of 60% or more, 65% or more, 70% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more or 99.9% or more to the specific nucleic acid sequence or amino acid sequence or essentially comprises thereof and maintains the original function and/or desired function.

According to one specific embodiment, the original function and/or desired function of the polynucleotide provided herein may be a function as a promoter. The polynucleotide may be operatively linked to a target gene as a promoter, and when linked, addition, and/or deletion, and/or substitution and the like of a nucleotide for use of restriction enzyme may be introduced at the 5' end and/or 3' end of the corresponding polynucleotide. The target gene may be a gene encoding a target protein, and the target protein may be involved in production of a target product as described above, and its examples is as follows.

In one embodiment, the protein involved in production of arginine among the amino acids may be at least one selected from proteins involved in arginine biosynthesis (arginine biosynthesis proteins). For example, the protein involved in arginine biosynthesis may be N-acetylglutamate synthetase (argA), N-acetylglutamate kinase (argB), N-acetylglutamyl phosphate reductase (argC), acetyl ornithine aminotransferase (argD), acetyl ornithine deacetylase (argE), ornithine carbamoyltransferase (argF, argl), argininosuccinate synthetase (argG), argininosuccinate lyase (argH), ornithine acetyltranferase (argJ), carbamoyl phosphate synthetase (carAB), but is not limited thereto.

In one embodiment, the protein involved in production of histidine among the amino acids may be at least one selected from proteins involved in histidine biosynthesis (histidine biosynthesis proteins). For example, the protein involved in histidine biosynthesis may be ATP phosphoribosyl transferase (hisG), phosphoribosyl-AMP cyclohydrolase (hisl), phosphoribosyl-ATP pyrophosphohydrolase (hisl), phosphoribosylformimino5-aminoimidazolecarboxamideribotideisomerase (hisA), amidotransferase (hisH), histidinol phosphate aminotransferase (hisC), histidinol phosphatase (hisB), histidinol dehydrogenase (hisD), but is not limited thereto.

In one embodiment, the protein involved in production of lysine among the amino acids may be at least one selected from proteins involved in lysine biosynthesis (lysine biosynthesis proteins). For example, the protein involved in lysine biosynthesis may be at least one selected from the group consisting of dihydrodipicolinate synthetase (dapA), aspartokinase III (lysC), dihydrodipicolinate reductase (dapB), diaminopimelate decarboxylase (lysA), diaminopimelate dehydrogenase (ddh), phosphoenolpyruvate carboxylase (ppc), aspartate semialdehyde dehydrogenase (asd), aspartate transaminase (aspC), diaminopimelate epimerase (dapF), tetrahydrodipicolinate succinylase (dapD), succinyl-diaminopimelate deacylase (dapE) and aspartase (aspA), but is not limited thereto.

In one embodiment, the protein involved in production of aspartic acid among the amino acids may be at least one selected from proteins involved in aspartic acid biosynthesis (aspartic acid biosynthesis proteins). For example, the protein involved in aspartic acid biosynthesis may be aspartate aminotransferase or the like, but is not limited thereto.

In one embodiment, the protein involved in production of glutamic acid among the amino acids may be at least one selected from proteins involved in glutamic acid biosynthesis (glutamic acid biosynthesis proteins). For example, the protein involved in glutamic acid biosynthesis may be at least one selected from the group consisting of glutamate dehydrogenase (gdhA), glutamine synthetase (glnA), glutamate synthetase (gltBD), isocitrate dehydrogenase (icdA), aconitate hydratase (acnA, acnB), citrate synthetase (gltA), methylcitrate synthetase (prpC), phosphoenolpyruvate carboxylase (ppc), pyruvate carboxylase (pyc), pyruvate dehydrogenase (aceEF, IpdA), pyruvate kinase (pykA, pykF), phosphoenolpyruvate synthetase (ppsA), enolase (eno), phosphoglyceromutase (pgmA, pgml), phosphoglyceric acid kinase (pgk), glyceraldehyde3-phosphate dehydrogenase (gapA), triosephosphate isomerase (tpiA), fructose bisphosphate aldolase (fbp), phosphofructokinase (pfkA, pfkB), glucose phosphate isomerase (pgi), 6-phosphogluconate dehydratase (edd), 2-kteo-3-deoxy-6-phosphogluconate aldolase (eda), transhydrogenase, or the like, but is not limited thereto.

In one embodiment, the protein involved in production of serine among the amino acids may be at least one selected from proteins involved in serine biosynthesis (serine biosynthesis proteins). For example, the protein involved in serine biosynthesis may be at least one selected from the group consisting of 3-phosphoglyceric acid dehydrogenase (serA), phosphoserine transaminase (serC), phosphoserine phosphatase (serB) or the like, but is not limited thereto.

In one embodiment, the protein involved in production of threonine among the amino acids may be at least one selected from proteins involved in threonine biosynthesis (threonine biosynthesis proteins). For example, the protein involved in threonine biosynthesis may be aspartokinase III (lysC), aspartate semialdehyde dehydrogenase (asd), aspartokinase I (thrA), homoserine kinase (thrB), threonine synthetase (thrC), aspartate aminotransferase, but is not limited thereto.

In one embodiment, the protein involved in production of asparagine among the amino acids may be at least one selected from proteins involved in asparagine biosynthesis (asparagine biosynthesis proteins). For example, the protein involved in asparagine biosynthesis may be transaminase, asparagine synthetase, or the like, but is not limited thereto.

In one embodiment, the protein involved in production of glutamine among the amino acids may be at least one selected from proteins involved in glutamine biosynthesis (glutamine biosynthesis proteins). For example, the protein involved in glutamine biosynthesis may be at least one selected from the group consisting of glutamate dehydrogenase (gdhA), glutamate synthetase (glnA), or the like, but is not limited thereto.

In one embodiment, the protein involved in production of cysteine among the amino acids may be at least one selected from proteins involved in cysteine biosynthesis (cysteine biosynthesis proteins). For example, the protein involved in cysteine biosynthesis may be at least one selected from the group consisting of serine acetyltransferase (cysE), 3-phosphoglycerate dehydrogenase (serA), or the like, but is not limited thereto.

In one embodiment, the protein involved in production of glycine among the amino acids may be at least one selected from proteins involved in glycine biosynthesis (glycine biosynthesis proteins). For example, the protein involved in glycine biosynthesis may be alanine-glyoxylate transaminase, or the like, but is not limited thereto.

In one embodiment, the protein involved in production of proline among the amino acids may be at least one selected from proteins involved in proline biosynthesis (proline biosynthesis proteins). For example, the protein involved in proline biosynthesis may be at least one selected from the group consisting of glutamate-5-kinase (proB), γ - glutamyl-phosphate reductase, pyrroline-5-carboxylate reductase (putA), and the like, but is not limited thereto. In one embodiment, the protein involved in production of alanine among the amino acids may be at least one selected from proteins involved in alanine biosynthesis (alanine biosynthesis proteins). For example, the protein involved in alanine biosynthesis may be alanine transaminase, or the like, but is not limited thereto.

In one embodiment, the protein involved in production of valine among the amino acids may be at least one selected from proteins involved in valine biosynthesis (valine biosynthesis proteins). For example, the protein involved in valine biosynthesis may be acetohydroxy acid isomeroreductase (IIvC), dihydroxy-acid dehydratase (IIvD), branched-chain amino acid aminotransferase (IIvE), but is not limited thereto.

In one embodiment, the protein involved in production of isoleucine among the amino acids may be at least one selected from proteins involved in isoleucine biosynthesis (isoleucine biosynthesis proteins). For example, the protein involved in isoleucine biosynthesis may be acetohydroxy acid synthetase (AHAS), acetohydroxy acid isomeroreductase, dihydroxy acid dehydratase, valine aminotransferase, or the like, but is not limited thereto.

In one embodiment, the protein involved in production of leucine among the amino acids may be at least one selected from proteins involved in leucine biosynthesis (leucine biosynthesis proteins). For example, the protein involved in leucine biosynthesis may be acetolactate synthase, acetohydroxy acid isomeroreductase, dihydroxy acid dehydratase, α -Isopropylmalic acid synthase, α - Isopropylmalate isomerase, leucine aminotransferase, or the like, but is not limited thereto.

In one embodiment, the protein involved in production of methionine among the amino acids may be at least one selected from proteins involved in methionine biosynthesis (methionine biosynthesis proteins). For example, the protein involved in methionine biosynthesis may be aspartic acid kinase, aspartic acid-semialdehyde dehydrogenase, homoserine dehydrogenase, homoserine O-succinyltransferase, cystathionine γ -generating enzyme, cystathionine β -lyase, methionine synthase, or the like, but is not limited thereto.

In one embodiment, the protein involved in production of phenylalanine among the amino acids may be at least one selected from proteins involved in phenylalanine biosynthesis (phenylalanine biosynthesis proteins). For example, the protein involved in phenylalanine biosynthesis may be chorismate mutase, prephenate aminotransferase, arogenate dehydratase, or the like, but is not limited thereto.

In one embodiment, the protein involved in production of tyrosine among the amino acids may be at least one selected from proteins involved in tyrosine biosynthesis (tyrosine biosynthesis proteins). For example, the protein involved in tyrosine biosynthesis may be chorismate mutase, prephenate aminotransferase, arogenate dehydrogenase, or the like, but is not limited thereto.

In one embodiment, the protein involved in production of tryptophane among the amino acids may be at least one selected from proteins involved in tryptophane biosynthesis (tryptophane biosynthesis proteins). For example, the protein involved in tryptophane biosynthesis may be anthranilate synthase, anthranilate phosphoribosyl transferase, anthranilate isomerase, imidazole glycerol phosphate synthase, tryptophan synthase, or the like, but is not limited thereto.

In one embodiment, the protein involved in production of O-acetyl homoserine among the amino acids may be at least one selected from proteins involved in O-acetyl homoserine biosynthesis. For example, the protein involved in O-acetyl homoserine biosynthesis may be homoserine O-acetyl transferase, or the like, but is not limited thereto.

In one embodiment, the protein involved in production of beta-alanine (β - alanine) among the amino acids may be at least one selected from proteins involved in beta-alanine biosynthesis. For example, the protein involved in beta-alanine biosynthesis may be propionate CoA ligase, medium chain acyl-CoA dehydrogenase, 3-hydroxypropionyl-CoA dehydratase, 3-hydroxypropionyl-CoA hydrolase, 3-hydroxypropionate dehydrogenase, beta alanine-pyruvate transaminase, or the like, but is not limited thereto.

The gene involved in production of the nucleic acid may be at least one or more genes selected from nucleic acid biosynthesis genes. For example, it may be at least one selected from the group consisting of amidophosphoribosyltransferase (purF), PRA-glycine ligase (purD), phosphoribosylaminoimidazolesuccinocarboxamide synthase (purC), bifunctional AICAR formyltransferase/IMP cyclohydrolase (purH), adenylosuccinate synthase (purA), adenylosuccinate lyase (purB), phosphoribosylaminoimidazole mutase (purE), phosphoribosylaminoimidazole carboxylase (purK), or the like, but is not limited thereto.

In one specific embodiment, the target gene may be at least one selected from the group consisting of pyc gene, metX gene, and ilvE gene.

According to one specific embodiment, the *pyc* gene encodes pyruvate carboxylase, and according to one specific embodiment, the polynucleotide and *pyc* gene are operatively linked, and may be used for production of L-threonine. The pyc gene may be a gene derived from Corynebacterium glutamicum.

According to one specific embodiment, the *metX* gene encodes homoserine O-acetyltransferase, and according to one specific embodiment, the polynucleotide and *metX* gene are operatively linked, and may be used for production for O-acetyl homoserine. The *metX* gene may be a gene derived from Corynebacterium glutamicum.

According to one specific embodiment, the ilvE gene encodes branched-chain amino acid aminotransferase, and according to one specific embodiment, the polynucleotide and ilvE gene are operatively linked, and may be used for production of valine. The ilvE gene may be a gene derived from Corynebacterium glutamicum.

Another aspect provides an expression cassette comprising the polynucleotide provided herein, and a target gene.

The target gene may be a gene encoding a target protein, and the target protein may be a protein (for example, enzyme) involved in production of a target product.

The target product may be at least one selected from the group consisting of amino acids, amino acid derivatives, nucleic acids, nucleic acid derivatives, vitamins, vitamin derivatives, sugars, sugar derivatives, fatty acids, fatty acid derivatives, proteins and other metabolites and the like.

The expression cassette may comprise the polynucleotide as a promoter.

In the present description, the term "expression cassette" may refer to a construct comprising at least a target gene and an expression regulatory sequence (for example, promoter), which is a nucleic acid fragment of the minimal unit capable of being expressed in a host cell.

The polynucleotide may be comprised at the 5' end or 3' end region of the target gene.

The polynucleotide may be operatively linked to the target gene.

The polynucleotide, target gene, target protein and target protein are as described above.

Other aspect provides a vector, comprising the polynucleotide provided herein or the expression cassette.

The vector may be a recombinant vector.

The recombinant vector may be an expression vector or insertion vector.

The vector may comprise the polynucleotide as a promoter.

According to one specific embodiment, the vector may comprise the polynucleotide having promoter activity and a target gene operatively linked to the polynucleotide. The target gene may be a gene encoding a target protein, and the target protein may be a protein (for example, enzyme) involved in production of a target product.

According to one specific embodiment, the target protein may be a fusion protein in which one protein (single protein), or 2 or more proteins are fused. When the target protein is a fusion protein comprising 2 or more proteins, the gene encoding target protein may be a fusion gene comprising genes encoding the 2 or more proteins, respectively.

According to one specific embodiment, when the gene encoding the target protein is a fusion gene comprising genes encoding 2 or more proteins, respectively, in the recombinant vector, it may be designed for all the genes comprised in the fusion gene to be under control of one polynucleotide (promoter), or for at least one of them to be under control of a separate polynucleotide. For example, the vector may comprise the polynucleotide having promoter activity and one gene or 2 or more (for example, 2, 3, 4, 5, 6, 7, 8, 9, or 10) genes operatively linked to the polynucleotide, and when 2 or more genes are comprised, it may comprise one polynucleotide having promoter activity (that is, that the 2 or more genes are under control of one promoter), or comprise two or more (in this case, the polynucleotide may be comprised less than the number of the genes so that at least one of the 2 or more genes is under control of a separate promoter.

In the present description, the term "vector" collectively refers to DNA molecules for delivering nucleic acids of a target sequence into an appropriate host or cell, and it may comprise an appropriate gene expression regulatory sequence; and optionally, a nucleic acid sequence of a target gene operatively linked thereto. The "gene expression regulatory sequence" is elements that perform various regulatory functions in gene expression, and in one embodiment, it may comprise the polynucleotide having promoter activity provided in the present description, and may refer to a nucleic aid sequence capable of expressing a target gene operatively linked thereto. Specifically, the gene expression regulatory sequence may comprise any operator sequence to regulate transcription, a sequence encoding an appropriate mRNA ribosome binding site, and/or a nucleic acid sequence that regulates termination of transcription and translation and the like, in addition to a promoter for transcription of the gene, but is not limited thereto. In addition, as a regulatory sequence suitable for prokaryotic organisms, a promoter and a ribosome binding site may be comprised, but is not limited thereto. Those skilled in the art may constitute the gene expression regulatory sequence comprising the polynucleotide having promoter activity of the present application as needed.

In the present description, "operatively linked" refers to that the polynucleotide having promoter activity is functionally linked to the nucleotide sequence of the target gene to initiate and/or mediate transcription of the target gene. Operative linking may be performed using a gene recombination technology known in the art, for example, site-specific DNA cleavage and/or linkage technologies, and the site-specific DNA cleavage and linkage may be performed using common lyase and/or ligase and the like, but is not limited thereto.

The vector is not particularly limited as long as it can be transformed into a host cell and/or expressed in the host cell. Examples of the vector may include plasmids, cosmids, viruses, and/or bacteriophages in a natural state or a recombinant state. For example, as the phage vector or cosmid vector, at least one selected from the group consisting of pWE15, M13, λLB3, λBL4, λIXII, λASHII, λAPII, λt10, λt11, Charon4A, Charon21A, and the like may be used, and as the plasmid vector, at least one selected from the group consisting of pBR-based, pUC-based, pBluescriptll-based, pGEM-based, pTZ-based, pCL-based, pET-based, and the like may be used.

In one embodiment, an endogenous promoter in chromosome of a host cell may be replaced with the polynucleotide having promoter activity provided in the present description. In this case, the vector may be a vector for inserting chromosome in a host cell comprising the polynucleotide having promoter activity, and for example, at least one selected from the group consisting of pECCG117, pDZ, pDC, pACYC177, pACYC184, pCL, pUC19, pBR322, pMW118, pCC1BAC, pCES208, pXMJ19 vectors and the like may be used, but is not limited thereto. In addition, insertion of the polynucleotide into chromosome may be performed by any method known in the art, for example, homologous recombination, genome editing by target-specific endonuclease (for example, RNA-guided endonuclease such as Cas9, Cpf1 and the like) and the like.

In one embodiment, when the vector or the polynucleotide having promoter activity comprised therein and/or target gene are inserted into chromosome, the vector may further comprise a selection marker for confirming chromosome insertion. As the selection marker, markers that give a selectable phenotype such as drug (e.g., antibiotic) resistance, auxotrophy, resistance to a cytotoxic agent, or expression of a surface protein may be used. In an environment treated with a selective agent (drug, cytotoxic agent, etc.), only cells expressing the selection marker survive or show other phenotypes, so cells in which a sequence related to the selection marker is inserted into chromosome may be selected.

The polynucleotide, target gene, target protein, target product and expression cassette are as described above.

Other aspect provides a host cell or recombinant cell, comprising at least one selected from the group consisting of the polynucleotide provided herein, the expression cassette and the vector.

In the present description, the term "recombinant cell" may refer to a transformant in which the polynucleotide and/or the recombinant vector are introduced. The term "transformation" means changing genetic traits of the host cell (microorganism) by introducing a target nucleic acid (DNA or RNA) into a host cell (microorganism). The transformation method may be performed by selecting an appropriate standard technology known in the art depending on the host cell. For example, the transformation includes electroporation, lipofection, calcium phosphate (CaPO₄) precipitation, calcium chloride (CaCl₂) precipitation, microinjection, polyethylene glycol (PEG) method, DEAE-dextran method, cation liposome method, and lithium acetate-DMSO method and the like, but is not limited thereto.

The host cell may include any one without limitations as long as it is a cell in which the polynucleotide provided herein operates as a promoter to allow (or increase) expression of a (operatively linked) target gene (gene comprised in the recombinant vector or host cell endogenous gene). In one embodiment, the host cell may be a microorganism, a plant cell, or an animal cell.

The microorganism (or strain) of the present application may be a microorganism which has target product production ability, or has improved (or increased) target product production ability.

The microorganism of the present application may be a microorganism naturally having no target product production ability, or a microorganism in which the target product production ability is given or improved, as at least one selected from the group consisting of the polynucleotide, the expression cassette and the vector is introduced into a microorganism having target product production ability, but is not limited thereto.

That the microorganism has improved target product production ability or has target product production ability may mean that the microorganism has improved target product production ability than a non-modified microorganism, a cell before recombination, a parent strain, and/or a wild-type strain, or is given the target product production ability differently from the non-modified microorganism cell before recombination, parent strain and/or wild-type strain, which have no target product production ability.

The microorganism in which at least one selected from the group consisting of the polynucleotide, the expression cassette and the vector is introduced may have improved target product production ability, compared to a microorganism before introduced or a microorganism before intensified, that is, a homogenous non-modified microorganism. Herein, "non-modified microorganism" does not exclude a strain which comprises a mutation that can occur naturally in the microorganism, and may mean a wild-type strain or a natural strain itself, or a strain before traits are changed by genetic mutation due to a natural or artificial factor. For example, the non-modified microorganism may refer to a strain in which at least one selected from the group consisting of the polynucleotide, the expression cassette and the vector is not introduced, or before it is introduced. The "non-modified microorganism" may be interchangeably used with "strain before modified", "microorganism before modified", "non-mutated strain", "non-modified strain", "non-mutated strain", "non-mutated microorganism" or "reference microorganism". The polynucleotide, the expression cassette and the vector are as described above.

In one specific embodiment, the microorganism may be for example, a microorganism of *Escherichia* sp., *Corynebacterium* sp., or *Bacillus* sp., or the like, and more specifically, it may be *Corynebacterium glutamicum,* or *Bacillus subtilis,* but is not limited thereto.

In one embodiment, the microorganism may be a microorganism of Corynebacterium genus, a microorganism of Escherichia genus, and/or a microorganism of Bacillus genus.

The microorganism of Corynebacterium genus may be at least one selected from the group consisting of *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium acetoacidophilum, Corynebacterium acetoglutamicum, Corynebacterium alkanolyticum, Corynebacterium lilium, Corynebacterium melassecola, Corynebacterium thermoaminogenes, Corynebacterium herculis* and *Corynebacterium flavescens,* but is not limited thereto.

The microorganism of Bacillus genus may be at least one selected from the group consisting of *Bacillus subtilis, Bacillus amyloliquefaciens, Bacillus methylotrophicus, Bacillus licheniformis,* Bacillus velezensis, *Bacillus sonorensis* and *Bacillus valismortis,* but is not limited thereto.

The microorganism of Escherichia genus may be *Escherichia coli,* but is not limited thereto. As one example, a microorganism into which at least one selected from the group consisting of the polynucleotide, the expression cassette and the vector is introduced, may be newly given a production ability of the target product, or have one increased by about 2% or more, about 3% or more, about 4% or more, about 5% or more, about 6% or more, about 7% or more, about 8% or more, about 9% or more, about 10% or more, about 11% or more, about 12% or more, about 13% or more, about 14% or more, about 15% or more, about 16% or more, about 20% or more, about 25% or more, about 30% or more, about 40% or more, about 50% or more, about 60% or more, about 70% or more, about 80% or more, about 90% or more, about 100% or more, about 150% or more, about 200% or more, about 250% or more, about 300% or more, about 400% or more, about 500% or more, about 600% or more, about 700% or more, about 800% or more, about 900% or more, about 1,000% or more, about 1,500% or more, about 2,000% or more, about 2,500% or more, or about 3,000% or more, compared to a parent strain before mutation, non-modified microorganism, parent strain, or wild-type microorganism, but is not limited thereto.

As another example, in the microorganism with the improved target product production ability, compared to the parent strain before mutation, and non-modified microorganism, the target product production ability may be about 1.02 times or more, about 1.03 times or more, about 1.04 times or more, about 1.05 times or more, about 1.06 times or more, about 1.07 times or more, about 1.08 times or more, about 1.09 times or more, about 1.1 times or more, about 1.11 times or more, about 1.12 times or more, about 1.13 times or more, about 1.14 times or more, about 1.15 times or more, about 1.16 times or more, about 1.2 times or more, about 1.3 times or more, about 1.4 times or more, about 1.5 times or more, about 1.6 times or more, about 1.7 times or more, about 1.8 times or more, about 1.9 times or more, about 2 times or more, about 2.5 times or more, about 3 times or more, about 4 times or more, about 5 times or more, about 6 times or more, about 7 times or more, about 8 times or more, about 9 times or more, about 10 times or more, about 15 times or more, about 20 times or more, about 25 times or more, or about 30 times or more (the upper limit is not particularly limited, and for example, it may be about 1,000 times or less), but is not limited thereto.

As other example, in the microorganism with the improved target product production ability, compared to the parent strain before mutation, and non-modified microorganism, the target product production ability may be about 0.1 g/L or more, about 0.2 g/L or more, about 0.3 g/L or more, about 0.4 g/L or more, about 0.5g/L or more, about 0.6 g/L or more, about 0.7 g/L or more, about 0.8 g/L or more, about 0.9 g/L or more, about 1g/L or more, about 1.1 g/L or more, about 1.2 g/L or more, about 1.3 g/L or more, about 1.4 g/L or more, about 1.5g/L or more, about 1.6 g/L or more, about 1.7 g/L or more, about 1.8 g/L or more, about 1.9 g/L or more, about 2.0g/L or more, about 2.5g/L or more, about 3g/L or more, about 3.5g/L or more, about 4g/L or more, about 4.5g/L or more, about 5g/L or more, about 5.5g/L or more, about 6g/L or more, about 7g/L or more, about 8g/L or more, about 9g/L or more, about 10g/L or more, about 15g/L or more, about 20g/L or more, about 25g/L or more, about 30g/L or more (the upper limit is not particularly limited, and for example, it may be about 100g/L or less), but is not limited thereto.

The term, "about" is a range including all of ±0.5, ±0.4, ±0.3, ±0.2, ±0.1 and the like, and includes all numerical values in the equivalent or similar range to the numerical value after the term, about.

The polynucleotide, the expression cassette and the vector are as described above.

Other aspect provides a composition for producing a target product comprising the microorganism, a medium in which the microorganism is cultured, or a combination thereof.

The composition may further comprise any appropriate excipient commonly used in the composition for producing a target product, and this excipient, may be, for example, a preservative, a wetting agent, a dispersant, a buffer, a stabilizer, or a tonicifying agent, but is not limited thereto.

Other aspect provides a use for using in production of a target product.

Other aspect provides a use for using in preparation of a composition for producing a target product.

Other aspect provides a method for production of a target product, comprising culturing the microorganism in a medium.

The method may further comprise recovering the target product from the medium or microorganism according to the culturing.

The medium in which the microorganism is cultured may comprise or not comprise the microorganism.

The microorganism may comprise at least one selected from the group consisting of the polynucleotide provided herein, an expression cassette comprising the polynucleotide and a target gene, and a vector comprising the expression cassette, as described above.

In the present description, "culture" refers to growing cells under an artificially controlled environmental condition. The method for production of a target product provided in the present description may be performed by appropriately selected means among all methods widely known in the art. For example, the culture may be continuously culturing in a batch process, a fed batch or repeated fed batch process, but is not limited thereto. The medium used for culturing may satisfy growth requirements of cells subjected to culturing in an appropriate method.

Herein, "medium" refers to a substance in which nutrients required for culturing the microorganism, for example, Corynebacterium glutamicum strain, are mixed as major components, and nutrients and growth factors and the like including water essential for survival and development are supplied. Specifically, for the medium used for the culturing of the microorganism of the present application and other culture condition, any one may be used without particular limitations, as long as it is a medium used for culturing a common microorganism, but the microorganism of the present application may be cultured in a common medium containing suitable carbon sources, nitrogen sources, phosphorus sources, inorganic compounds, amino acids, and/or vitamins, while adjusting the temperature, pH and the like under an aerobic condition.

Specifically, the culture medium for the microorganism of the present application, for example, the microorganism of Corynebacterium genus may be found in the document ["Manual of Methods for General Bacteriology" by the American Society for Bacteriology (Washington D.Corynebacterium, USA, 1981)].

Sugar sources which may be used for the culturing or comprised in the medium may comprise at least one selected from the group consisting of sugars and carbohydrates such as glucose, saccharose, lactose, fructose, maltose, starch, cellulose and the like; oils and fats such as soybean oil, sunflower oil, castor oil, coconut oil and the like; fatty acids such as palmitic acid, stearic acid, and linoleic acid; alcohols such as glycerol, ethanol, and the like; organic acids such as acetic acid, and the like, but are not limited thereto. Nitrogen sources which may be used for the culturing or comprised in the medium, may include at least one selected from the group consisting of organic nitrogen sources such as peptone, yeast extracts, meat juice, malt extracts, corn steep liquor, soybean meal, urea and the like, and inorganic nitrogen sources such as ammonium sulfate, ammonium chloride, ammonium phosphate, ammonium carbonate, ammonium nitrate and the like, but are not limited thereto. Phosphorus sources which may be used for the culturing or comprised in the medium, may comprise at least one selected from the group consisting of potassium salts of phosphate such as potassium dihydrogen phosphate, dipotassium hydrogen phosphate and the like and sodium salts corresponding thereto, and the like, but are not limited thereto. In addition thereto, the medium may contain metal salts such as magnesium sulfate or iron sulfate required for growth. Furthermore, in addition thereto, the culturing or medium may further use or comprise at least one selected from essential growth substances such as amino acids and vitamins. Moreover, the medium may comprise an appropriate precursor of the target product as a raw material. The raw materials described above may be added to the culture in batches and/or continuously in the culturing process.

During the culturing of cells, using base compounds such as sodium hydroxide, potassium hydroxide and ammonia, and/or acid compounds such as phosphate or sulfate in an appropriate manner, the pH of the culture may be adjusted. In addition, during the culturing, using an antifoaming agent such as fatty acid polyglycol ester and the like, foam generation may be inhibited. Furthermore, in order to maintain the **aerobic** condition, oxygen or oxygen-containing gas (e.g., air) may be injected into the culture. The temperature of the medium and/or culture may be commonly 20°C to 45°C, or 25°C to 40°C. The culturing time may continue until the production of the target product reaches the desired amount, and for example, it may be about 10 to about 160 hours, but is not limited thereto.

The isolating or recovering the target substance from the cultured microorganism or culture medium may be performed using an appropriate method known in the art depending on the culturing method. For example, a method such as centrifugation, filtration, extraction, spray, drying, evaporation, precipitation, crystallization, electrophoresis, fractional dissolution (for example, ammonium sulfate precipitation), and/or chromatography (for example, ion exchange, affinity, hydrophobic and size exclusion) and the like, but is not limited thereto. The culturing medium refers to a medium in which recombinant cells are cultured.

According to one specific embodiment, the isolating or recovering the target product may be isolating supernatant obtained by low-speed centrifuging the culture to remove biomass through ion exchange chromatography.

The method for producing the target product may further comprise a process of purifying the target product.

The recovering the target product from the culture (medium) may be performed by an isolation method by common means known in the art. As the common means which may be used for isolating of the target product, centrifugation, filtration, chromatography and/or crystallization methods and the like may be exemplified. In one embodiment, the target product may be isolated by performing ion exchange chromatography for the supernatant obtained by low-speed centrifuging the culture to remove biomass, but is not limited thereto. The recovering may further comprise a purification process.

### [ADVANTAGEOUS EFFECTS]

The polynucleotide according to one specific embodiment has a promoter activity, and is introduced into a microorganism, so it can increase expression and activity of a gene operatively linked thereto, and it can be usefully used for efficiently producing a target product affected by the polynucleotide and gene.

### [MODE FOR INVENTION]

Hereinafter, the present invention will be described in more detail by the following examples. However, they are intended to illustrate the present invention only, but the scope of the present invention is not limited by these examples.

### Example 1. Construction of recombinant vectors using novel promoters

Various kinds of promoter sequences of Corynebacterium sp. microorganisms and Escherichia sp. microorganisms were analyzed, and polynucleotides consisting of a novel sequence of SEQ ID NO: 1, SEQ ID NO: 2 or SEQ ID NO: 3 predicted to show the strong activity of inducing expression were synthesized therefrom, and named Psba, Psbb, Psbc, respectively.

In order to confirm the promoter activity of the polynucleotides, recombinant vectors expressing eGFP (enhanced green fluorescent protein) regulated by the Psba, Psbb, Psbc or conventionally known Pcj7 promoter (U.S. Granted Patent US 7662943 B, SEQ ID NO: 15), and the fluorescence sensitivity thereof was compared.

At first, using pCES208 (J.Microbiol. Biotechnol., 18:639-647, 2008) vector, a E. coli-Corynebacterium shuttle vector, an expression vector comprising the eGFP gene was prepared. The nucleic acid sequence of the eGFP gene was obtained from NIH nucleic acid sequence database of Genbank (GenBank: MN832871.1), and using the gene synthesis service of Cosmogenetech, the ORF of the eGFP gene was amplified. Using the eGFP gene as a template, using the primer sequence of SEQ ID NOs: 5 and 6, PCR was performed. PCR was performed by denaturation at 94°C for 5 minutes, and then repeating denaturation at 94°C for 30 seconds, annealing at 60°C for 30 seconds, and polymerization at 72°C for 30 seconds, 30 times, and then polymerization at 72°C for 5 minutes.

The amplified eGFP fragments were cloned into pCES208 vector cut with restriction enzyme EcoRV through Gibson Assembly method (DG Gibson et al., NATURE METHODS, VOL.6 NO.5, MAY 2009, NEBuilder HiFi DNA Assembly Master Mix). The recombinant vector was named pCES208-eGFP.

In order to introduce each promoter into the eGFP expression vector, using Pcj7 promoter (SEQ ID NO: 15), synthesized Psba (SEQ ID NO: 1), Psbb (SEQ ID NO: 2), Psbc (SEQ ID NO: 3) fragments as a template, using the primer sequences of SEQ ID NOs: 7 and 8, SEQ ID NOs: 9 and 10, SEQ ID NOs: 11 and 12, SEQ ID NOs: 13 and 14, respectively, PCR was performed. PCR was performed by denaturation at 94°C for 5 minutes, and then repeating denaturation at 94°C for 30 seconds, annealing at 60°C for 30 seconds, and polymerization at 72°C for 30 seconds, 30 times, and then polymerization at 72°C for 5 minutes.

Each of the amplified fragments was cloned into pCES208 vector cut with restriction enzyme EcoRV through Gibson Assembly method (DG Gibson et al., NATURE METHODS, VOL.6 NO.5, MAY 2009, NEBuilder HiFi DNA Assembly Master Mix), and they were named pCES208-Pcj7-eGFP, pCES208-Psba-eGFP, pCES208-Psbb-eGFP and pCES208-Psbc-eGFP, respectively.

### Example 2. Construction of recombinant strains comprising the novel promoter

The vectors prepared in Example 1 were introduced to Corynebacterium glutamicum ATCC13032 and ATCC13869 using electroporation (Appl. Microbiol. Biotechnol. (1999) 52: 541-545), respectively. After that, the transformed strains were selected in a Luria-Bertani (LB) medium comprising kanamycin 25 mg/l. Recombinant strains selected from Corynebacterium glutamicum ATCC13032 were named ATCC13032/pCES-eGFP, ATCC13032/pCES-Pcj7-eGFP, ATCC13032/pCES-Psba-eGFP, ATCC13032/pCES-Psbb-eGFP, and ATCC13032/pCES-Psbc-eGFP, respectively, and recombinant strains selected from Corynebacterium glutamicum ATCC13869 were named ATCC13869/pCES-eGFP, ATCC13869/pCES-Pcj7-eGFP, ATCC13869/pCES-Psba-eGFP, ATCC13869/pCES-Psbb-eGFP, and ATCC13869/pCES-Psbc-eGFP, respectively.

### Example 3. Evaluation of activity to induce eGFP expression of the novel promoter

The recombinant strain prepared in Example 2 was cultured as described below, and the eGFP activity of the recombinant strain was measured. Experiments were performed three times repeatedly, and mean values were shown in Table 1.

The recombinant strain was inoculated in a flask comprising the cultured solution of 25 ml, respectively, and cultured in a shaking incubator at 30°C for 24 hours.

### Glucose medium (pH 7.2)

Glucose 20 g, ammonium sulfate 5 g, yeast extract 5 g, urea 1.5 g, KH₂PO₄ 4 g, K₂HPO₄ 8 g, MgSO₄ 7 & H₂O 0.5 g, biotin 150 µg, thiamine hydrochloride 1.5 mg, calcium pantothenate 3 mg, nicotinamide 3 mg (based on 1 L distilled water)

Microbial cells were collected through centrifugation (5,000 rpm, 15 minutes) from the cultured solution, and washed with 50 mM Tris-HCl (pH 8.0) buffer solution, and then resuspended in the same buffer solution. Glass beads of 1.25 g per 1.5 ml of suspension were added, and then the microbial cells were disrupted using a bead beater for 6 minutes, and the supernatant was collected through centrifugation (15,000 rpm, 20 minutes), and the protein concentration by Bradford method (Bradford, M.M 1976. Anal. Biochem. 72:248-254) was quantified. Excitation light was irradiated at 488 nm using the method of Laure Gory et al. (FEMS Microbiology Letters 194, 127-133, 2001) and emission light was measured at 511 nm using a spectrophotometer (Perkin-Elmer) device, for the same amount of microbial cell extracts, to measure the expression level of the eGFP gene.

**[Table 1]**

| Strain | Promoter | Fluorescence sensitivity | Relative fluorescence sensitivity (%) |
|---|---|---|---|
| ATCC13032/pCES208-eGFP | Negative control | 9846.8 | 16.6 |
| ATCC13032/pCES208-Pcj7-eGFP | CJ7 | 59292.3 | 100 |
| ATCC13032/pCES208-Psba-eGFP | SBA | 107829.8 | 181.9 |
| ATCC13032/pCES208-Psbb-eGFP | SBB | 102510.9 | 172.9 |
| ATCC13032/pCES208-Psbc-eGFP | SBC | 61353.4 | 103.5 |
| ATCC13869/pCES208-eGFP | Negative control | 8233.6 | 18.2 |
| ATCC13869/pCES208-Pcj7-eGFP | CJ7 | 45350.5 | 100 |
| ATCC13869/pCES208-Psba-eGFP | SBA | 95715.8 | 211.1 |
| ATCC13869/pCES208-Psbb-eGFP | SBB | 71801.1 | 158.3 |
| ATCC13869/pCES208-Psbc-eGFP | SBC | 48266.2 | 106.4 |

As could be confirmed in Table 1 above, all the Psba, Psbb, Psbc promoters showed the promoter activity in the Corynebacterium glutamicum strain, and showed significantly higher eGFP fluorescence sensitivity than the Pcj7 promoter conventionally known as a strong promoter of Corynebacterium glutamicum.

In order to confirm whether the carbon source of the medium affected the promoter activity, the recombinant strain of Example 2 was cultured in a medium comprising sucrose instead of glucose, and an experiment was performed by the same method as above, and then the result was shown in Table 2 below.

### Sucrose medium (pH 7.2)

Sucrose 20 g, ammonium sulfate 5 g, yeast extract 5 g, urea 1.5 g, KH₂PO₄ 4 g, K₂HPO₄ 8 g, MgSO₄ 7 & H₂O 0.5 g, biotin 150 µg, thiamine hydrochloride 1.5 mg, calcium pantothenate 3 mg, nicotinamide 3 mg (based on 1 L distilled water)

**[Table 2]**

| Strain | Promoter | Fluorescence sensitivity | Relative fluorescence sensitivity (%) |
|---|---|---|---|
| ATCC13032/pCES208-eGFP | Negative control | 7520.4 | 11.6 |
| ATCC13032/pCES208-Pcj7-eGFP | CJ7 | 65034.1 | 100 |
| ATCC13032/pCES208-Psba-eGFP | SBA | 124712.4 | 191.8 |
| ATCC13032/pCES208-Psbb-eGFP | SBB | 86153.3 | 132.5 |
| ATCC13032/pCES208-Psbc-eGFP | SBC | 68229.2 | 104.9 |
| ATCC13869/pCES208-eGFP | Negative control | 6934.7 | 14.5 |
| ATCC13869/pCES208-Pcj7-eGFP | CJ7 | 47706.9 | 100 |
| ATCC13869/pCES208-Psba-eGFP | SBA | 85074.8 | 178.3 |
| ATCC13869/pCES208-Psbb-eGFP | SBB | 73924.2 | 155.0 |
| ATCC13869/pCES208-Psbc-eGFP | SBC | 54265.9 | 113.7 |

As could be confirmed in Table 2 above, the Psba, Psbb, Psbc promoters showed significantly higher eGFP fluorescence sensitivity than the Pcj7 promoter.

This result shows that the Psba, Psbb, Psbc promoters are strong promoters which induce expression of a target gene, regardless of the type of the microorganism introduced and carbon source of the medium.

From the above description, those skilled in the art to which the present invention pertains will understand that the present invention can be implemented in other specific forms without changing its technical spirit or essential features. In this regard, the examples described above should be understood as illustrative and not restrictive in all aspects. The scope of the present invention should be interpreted as all changed or modified forms derived from the meaning and scope of the claims described below and equivalent concepts thereof are included in the scope of the present invention.

## Claims

1. A polynucleotide comprising any one nucleic acid sequence selected from the group consisting of SEQ ID NO: 1, SEQ ID NO: 2 and SEQ ID NO: 3.

2. The polynucleotide according to claim 1, wherein the polynucleotide has a promoter activity.

3. An expression cassette comprising the polynucleotide of claim 1 and a target gene.

4. A microorganism comprising the polynucleotide of claim 1.

5. The microorganism according to claim 4, wherein the microorganism produces a target product.

6. The microorganism according to claim 5, wherein the target product is at least one selected from the group consisting of amino acid, amino acid derivative, nucleic acid, nucleic acid derivative, vitamin, vitamin derivative, protein, fatty acid, fatty acid derivative, organic acid and other metabolite.

7. The microorganism according to claim 4, wherein the microorganism is a microorganism of Corynebacterium genus, a microorganism of Escherichia genus, and/or a microorganism of Bacillus genus.

8. A method for production of a target product, comprising culturing the microorganism of any one of claims 4 to 7 in a medium.

9. The method for production of a target product according to claim 8, further comprising recovering the target product from the medium or microorganism according to the culturing.

10. The method for production of a target product according to claim 8, wherein the target product is at least one selected from the group consisting of amino acid, amino acid derivative, nucleic acid, nucleic acid derivative, vitamin, vitamin derivative, protein, fatty acid, fatty acid derivative, organic acid and other metabolite.

11. The method for production of a target product according to claim 8, wherein the microorganism is a microorganism of Corynebacterium genus, a microorganism of Escherichia genus, or a microorganism of Bacillus genus.
